# EUROPEAN PATENT SPECIFICATION

(11) **EP 2 922 459 B1**
(45) Date of publication and mention of the grant of the patent: **24.08.2016**
(21) Application number: 14703632.1
(22) Date of filing: 16.01.2014
(51) Int. Cl.: A61B 1/00, A61B 1/005

(54) **ENDOSCOPE COMBINED DEFLECTION CONTROL AND LOCK**
KOMBINIERTE DEFLEKTIONSSTEUERUNG UND SPERRUNG FÜR EIN ENDOSKOP
COMMANDE D'ENDOSCOPE COMBINANT VERROUILLAGE ET FLÉCHISSEMENT

(30) Priority: 22.01.2013 US 201313746683
(43) Date of publication of application: 30.09.2015
(73) Proprietor: Gyrus ACMI, Inc., Southborough, MA 01772 (US)
(72) Inventor: KONSTORUM, Gregory S., Stamford, CT 06902 (US)
(74) Representative: Harrison, Scott David
(86) International application number: PCT/IB2014/058341
(87) International publication number: WO 2014/115068

(56) References cited:
- EP-A1- 1 832 224
- EP-A1- 2 123 211
- WO-A2-2008/045374
- US-A1- 2012 078 050

## Description

### BACKGROUND OF THE INVENTION

### Field of the Invention

The invention relates to an endoscope and, more particularly, to a combined deflection control and lock.

### Brief Description of Prior Developments

There are flexible endoscopes with steering distal tip control mechanisms with separate deflection control and lock (or brake) levers. A disadvantage of this construction is that operation of a deflection control and a separate lock or brake requires using two hands or fingers, or using one finger with at least two steps (a lock/unlock step and a deflection step). For example, U.S. Patent No. 6,780,151 B2 discloses an endoscope with a control lever 17 and a separate brake lever 22. After the control lever 17 is moved by a finger, such as a thumb, the brake 22 can be moved by the same finger or a finger of another hand.

Document WO-A-2008/045374 relates to an endoscope comprising a lever to control the deflection of the distal end.

There is a desire to provide an endoscope deflection control and lock which can be operated by a single finger of a user.

### SUMMARY

The following summary is merely intended to be exemplary. The summary is not intended to limit the scope of the claimed invention.

In accordance with one aspect of the invention, an endoscope is provided according to claim 1.

In accordance with another aspect of the invention, an endoscope is provided according to claim 8.

In accordance with another aspect of the invention, a method of operating an endoscope according to claim 14 is provided

### BRIEF DESCRIPTION OF THE DRAWINGS

The foregoing aspects and other features of the invention are explained in the following description, taken in connection with the accompanying drawings, wherein:
Fig. 1 is a side view of an endoscope comprising features of the invention;
Fig. 2 is a perspective view of the endoscope shown in Fig. 1;
Fig. 3 is an enlarged cross sectional view of a portion of the endoscope shown in Figs. 1-2;
Fig. 4 is a top plan view of a portion of the endoscope shown in Figs. 1 - 2;
Fig. 5 is a perspective view of the lock/brake pad shown in Fig. 3;
Fig. 6 is a top plan view of the pad shown in Fig. 5;
Fig. 7 is a cross sectional view of the pad shown in Fig. 6 taken along line 7 - 7;
Fig. 8 is a perspective view of an alternate embodiment of the pad shown in Figs. 5 - 7;
Fig. 9 is a perspective view of an alternate embodiment of the frame piece shown in Fig. 3 showing an integrally formed lock/brake pad;
Fig. 10 is a partial perspective view of an alternate embodiment not part of the invention;
Fig. 11 is a cross sectional view of some of the components shown in Fig. 10; and
Fig. 12 is a perspective view of another example embodiment.

### DETAILED DESCRIPTION OF EMBODIMENTS

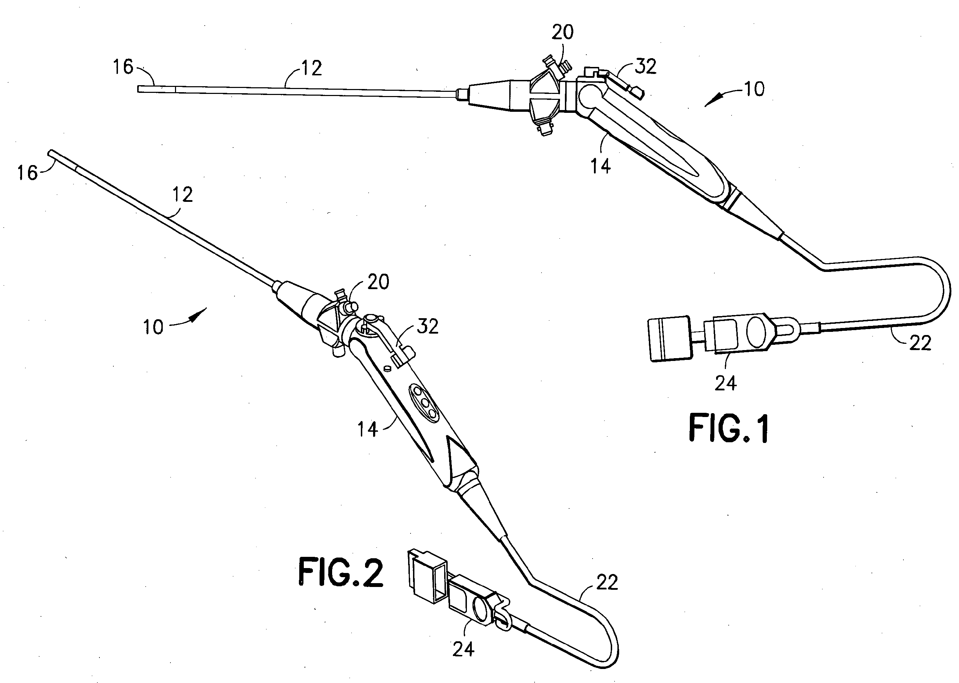

Referring to Figs. 1 and 2, an endoscope 10 is shown incorporating features of the invention. Although the invention will be described with reference to the example embodiments shown in the drawings, it should be understood that the invention can be embodied in many alternate forms of embodiments. In addition, any suitable size, shape or type of elements or materials could be used.

The endoscope 10, in this embodiment, is a hysteroscope used for inspection of the uterine cavity of a patient. The hysteroscope is an optical instrument connected to a video unit, and comprises channels for delivery and removal of a distention medium and a tool. However, features of the invention could be used in any suitable type of endoscope. In the example shown, the endoscope comprises a shaft 12 and a main section 14 which forms a handle. The shaft 12 has a front or distal end 16 which is controllably deflectable. In this embodiment the rest of the shaft is substantially rigid. The distal end 16 is adapted to deflection in left and right directions. However, any suitable deflection capability could be provided.

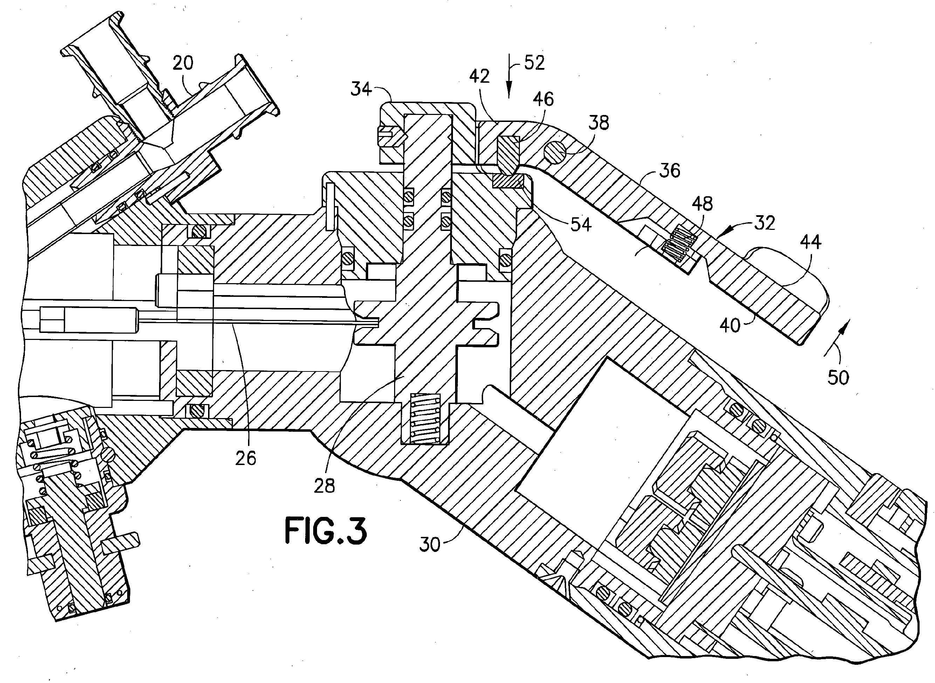

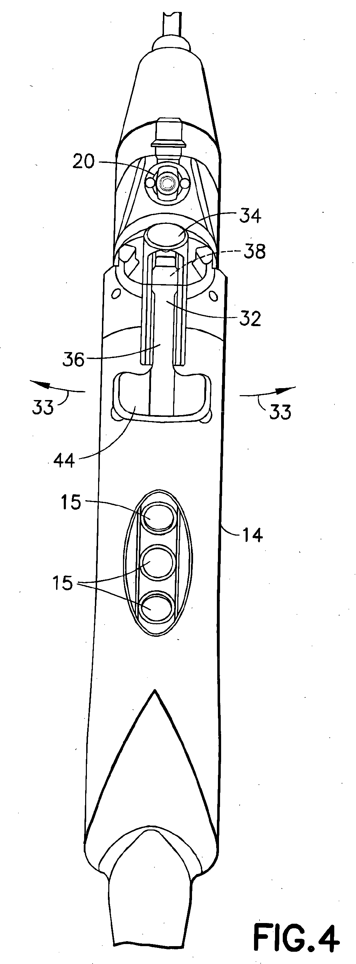

The main section 14 is located at the proximal end of the shaft 12. Referring also to Fig. 3, the main section 14 has a working instrument/irrigation inlet 20, and a cable 22 with a connector 24 adapted to be connected to another device. An electrical cable extends from light emitting diodes (LEDs) in the distal end 16 to the main section 14. Alternatively, an optical cable for illuminating in front of the distal end 16 could be provided. Deflection control wires or cables 26 extend from the distal end 16 to a pulley 28 in the main section 14. The pulley 28 is rotatably mounted in the frame 30 of the main section 14. As seen in Fig. 4, in this embodiment the main section 14 has buttons 15 to control features of another device which the connector 24 is connected to.

The pulley 28 and wires 26 form part of a deflection control for a user to control deflection of the deflectable distal end 16 from the main section 14. The deflection control also comprises a lever assembly 32. The lever assembly 32 is connected to the pulley 28 to axially rotate the pulley 28 when the lever assembly 32 is rotated right or left as indicated by arrows 33 in Fig. 4. The lever assembly 32 comprises a first lever 34 and a second lever 36. The first lever 34 is fixedly connected to the pulley 28. The second lever 36 is pivotably connected to the first lever 34 by a pivot pin 38. Thus, the second lever 36 can pivot on the first lever 34, and the second lever 36 is connected to the pulley 28 by the first lever 34; in series. However, any suitable movable connection of the second lever on the first lever could be provided.

The second lever 36 has a first end 40 and a second end 42 on opposite sides of the pivot pin 38. Referring also to Fig. 3, the first end 40 forms a finger receiver area 44 sized and shaped to receive a finger of a user, such as a thumb. The second end 42 has a lock/brake 46. In this embodiment the lock/brake 46 is a pin attached to the second end 42. However, any suitable lock/brake could be provided. A spring 48 is provided between the first and second levers 34, 36 to bias the second end 42 upward (see arrow 50) and the first end 40 downward (see arrow 52) on the first lever 34. However, a user can use his/her thumb to depress the first end 40 (reverse to direction 50) to thereby pivot the second lever 36 at the pivot pin 38 and move the second end 42 upward (reverse to direction 52).

The main section 14 includes a lock pad 54 located opposite the lock/brake 46. In this embodiment the pad 54 is an insert which is fixedly inserted into a receiving area of the main section. The insert has a general curved or arched shape. However, in an alternate embodiment the lock pad could be integrally formed with the frame of the main section, or any suitably sized and shaped lock/brake section (for engaging with the lock/brake 46 of the lever assembly) could be provided.

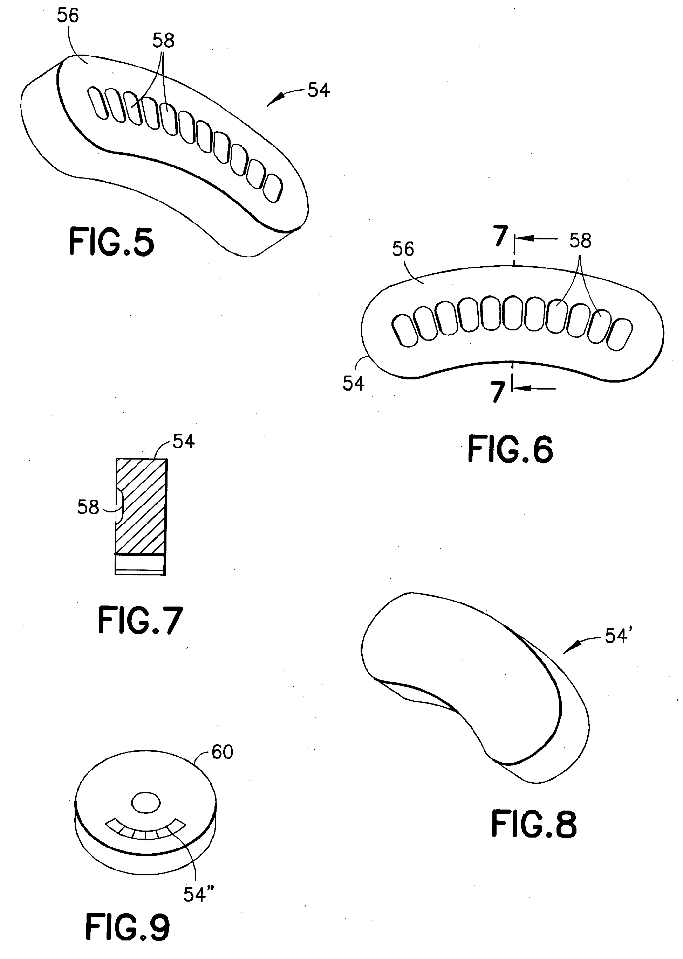

Referring also to Figs. 5-7, the pad 54 comprises a top side 56 having a series of recesses 58. The recesses 58 are sized, shaped and arranged in an arch or curve to receive the bottom tip of the pin 46 at different angular positions of the lever assembly 32. The recesses 58 allow positive location of the pin 46, and, thus, positive location of the angular position of the lever assembly 32, at one of a plurality of fixed predetermined locations selected by the user. When the second lever 36 is at its home position (with the second end 42 biased downward by the spring 48), the pin 46 is biased into one of the recesses 58. This prevents the lever assembly 32 from rotating relative to the main section 14 and, thus, prevents the lever assembly 32 from rotating the pulley 28. This forms a lock or brake for the deflection control system; fixing the position or shape of the distal end 16 on the shaft 12. The pin 46 forms a first lock/brake section which engages with a second lock/brake section formed by the pad 54.

With the same hand holding the handle formed by the main section 14, the user can use his or her thumb to depress the first end 40 of the second lever 36 (in a direction reverse to direction 50) to thereby disengage the pin 46 from the lock pad 54. The user is then free to swing the lever assembly 32 (both levers 34, 36 moving together in unison) left or right as indicated by arrows 33. Once a desired deflection of the distal end 16 is obtained, the user can release the first end 40 of the second lever 36 and the spring 48 can automatically biased the second lever 36 back to its home position. This automatically locks the lever assembly at a fixed angular position on the main section 14 due to the re-engagement of the lock/brake sections 46, 54.

With the invention, a construction can be provided which has an integrated deflection control and lock (brake) lever with an ergonomic design, and the lever can be activated with one finger (by a left of right hand person) eliminating the need of using two hands or fingers, or using two steps with a single finger.

With the example embodiment of the invention described above, the control lever 34 is firmly attached, such as with screws, to the external part of a pulley or pull cable mechanism 28. The lock (brake) lever 36 is pivotably attached to the control lever 34. A pin lock 46 is attached to one side of the lock lever, and a compression spring is placed between both levers at the other side of the lock lever; with the pivot pin in between. The lock insert 54 is placed and secured to the top surface of the endoscope enclosure/housing. The insert can be made from different materials and configurations.

Due to the location of the lock pin, the compression spring and the pivot pin, the lock pin creates a compression force to the lock pad/insert and the control lever is locked. In the example construction, the combined lever is always in the lock mode until positively moved by the user. In order to deflect the distal end 16 of the endoscope, the proximal end of the lock lever is pushed down first with a thumb (for the shown embodiment) to disengage the lock pin from the lock pad. Then, the combined lever assembly can be rotated with the thumb to the right or left causing the distal tip to deflect accordingly; dependent on the way the pull cables or wires attached to the pulley.

The lock pad insert can be made from rubber with brake action or from metal with indents with clicking action or holes for lock action. Referring also to Fig. 8, an alternate embodiment of the main section's lock pad 54' is shown without recesses, such as when the pad is made of rubber. Referring also to Fig. 9, an alternate embodiment is shown where the lock pad 54'' is made of metal integral with the frame piece 60. The pin 46 is preferably metal, but could be made of rubber or polymer material or could be integrally formed with the second lever.

A method of manufacturing an endoscope can be provided comprising:
▪ connecting a first lever to a control wire pulley of a deflection control system of the endoscope, wherein the lever is adapted to be rotated to rotate the pulley; and
▪ connecting a second lever to the first lever, wherein the second lever is adapted to pivot on the first lever.

The method can further comprise locating a spring between the first and second levers to bias the second lever towards a locked position. The method can further comprise biasing a lock section of the second lever against a lock section of a handle of the endoscope, wherein movement of the second lever on the first lever from a first position to a second position disengages engagement of the lock sections.

A method of operating an endoscope can be provided comprising
▪ rotating a finger lever in a first direction from a first locked position to a second unlocked position, wherein the finger lever is pivotably connected to a control lever, and wherein the control lever is connected to a control wire pulley of a deflection control system of an endoscope; and
▪ after the finger lever is moved to the second unlocked position, moving the finger lever in a second direction to thereby rotate the control lever and control wire pulley in the second direction.

The method can further comprise, after the finger lever and control lever are rotated in the second direction to a user selected location, allowing a spring to pivot the finger lever on the control lever in a third direction opposite to the first direction such that a lock section of the finger lever engages a lock section on a handle of the endoscope.

One of the features of the invention is the spring loaded control lever subassembly. In the embodiment shown in the drawings, the subassembly comprises the two levers 34, 36, spring 48 and pin 46. One of the features of the invention is a locking/breaking assembly having the pin 46 on the lever 36 and the engaged pad 54 on the frame 30; which can be used with only one hand/finger operation. The engagement between the pin and pad can be (for example) frictional engagement, positive locking engagement (such as a pin being located into a hole), and/or a detent or ratchet type of relative position locating system.

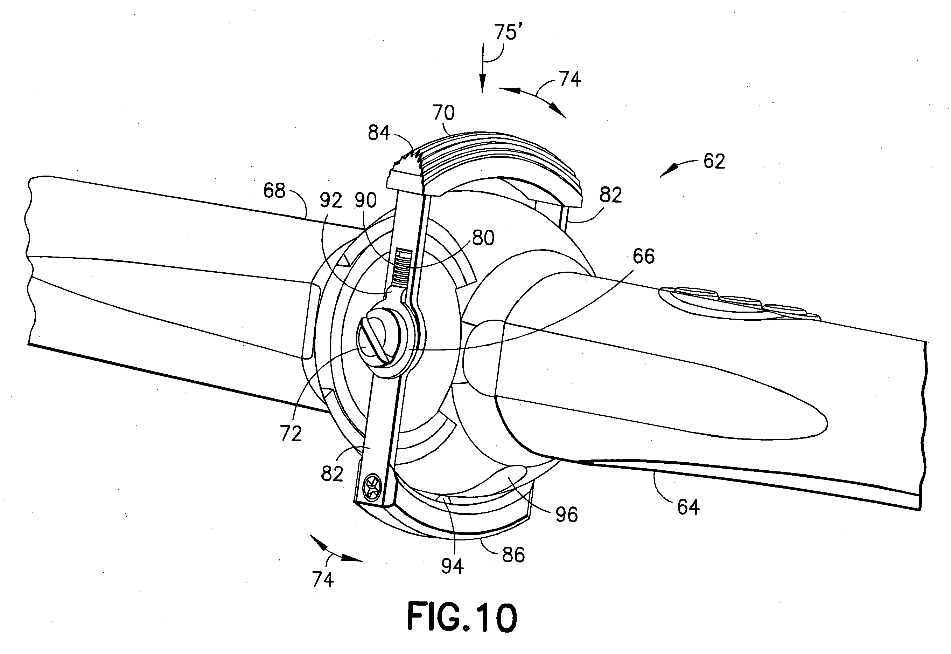

Referring now to Fig. 10, an alternate embodiment of the invention is shown. In this example embodiment rather than having the front distal end 16 which is configured to deflect horizontally left and right, the endoscope 62 has a front distal end which is configured to deflect vertically up and down. Rather than having the vertically orientated pulley 28, the main section 64 has a vertically orientated pulley with external ends 66 which are located at lateral sides of the frame 68. The pulley and deflection control wires extending up to the front distal end form part of a deflection control for a user to control deflection of the deflectable front end from the main section 14. The deflection control also comprises a lever assembly 70. The lever assembly 70 is connected to the external ends 66 of the pulley by screws 72 to axially rotate the pulley when the lever assembly 70 is rotated forward or backward as indicated by arrows 74 in Fig. 10.

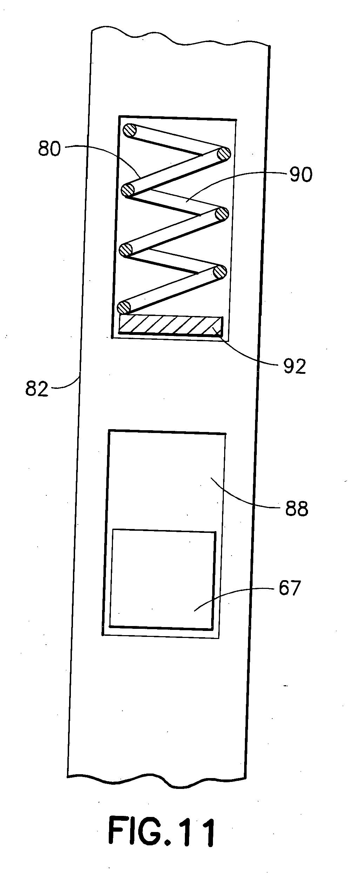

The lever assembly 70 comprises a lever member 76 and a spring 80. The lever member 76 has two arms 82 located on opposite lateral sides of the frame 68. Top and bottom sections 84, 86 connect the two arms 82 to each other. As seen in Fig. 11, each arm 82 has an elongate slot 88 at its center. At least one of the arms 82 also has a spring receiving pocket 90. In this example embodiment, square end sections 67 of the pulley shafts are slidably located in the slots 88. A portion 92 of the external end 66 of the pulley is located in the pocket 90. The spring 80 is located in the pocket 90 and biases the portion 92 towards the bottom of the pocket 90. More specifically, the spring 80 biases the lever member 76 upward as indicated by arrow 75.

The bottom section 86 has a brake/lock pin 94. The frame 68 has a brake/lock pad or pin engaging area 96. When the lever member 76 is biased upward by the spring (s) 80, this biases the pin 94 into a brake or lock position with the pad 76 to prevent rotation of the lever member 76 and, thus, prevent rotation of the deflection control wire pulley. In order to actively deflect the front distal end of the shaft up or down, the user can push the top section 84 downward as indicated by arrow 75'. Slots 88 provide clearance for the arms 82 relative to the screws 72. The spring(s) 80 is compressed in pocket(s) 90. The user can then rotate the lever member 76 backward or forward as indicated by arrow 74. Once the desired deflection of the front distal end is obtained, the user can release the lever member 76, and the spring (s) 80 can bias the lever member back to an upward home position. The pin 94 engages the pad 96 again to lock or frictionally engage the lever assembly 70 with the frame 68 again and thereby retain the distal front end at its deflected configuration/position.

A single finger of a user can be used to unlock the locking engagement of the pin 94 and pad 96 (by pressing down 75' on top section 84), rotate the deflection control wire pulley with the same finger (by rotating the tops section 84 forward or backward), and re-engage the locking engagement of the pin 94 and pad 96 (by releasing the finger from the top section 84 and allowing the springs(80) to bias the lever member 76 back to it home position). The lever assembly 70 can provide a combined lock and deflection control member at the handle, wherein the lever assembly 70 (combined lock and deflection control member) is adapted to be moved to a selected location by a single finger of a user and thereby deflect the distal end, and wherein the control member is adapted to be locked at a user selected location by movement of the lever assembly 70 (combined lock and deflection control member) to a locked position by the single finger of the user (in this case the single finger releasing depression of the lever member 76).

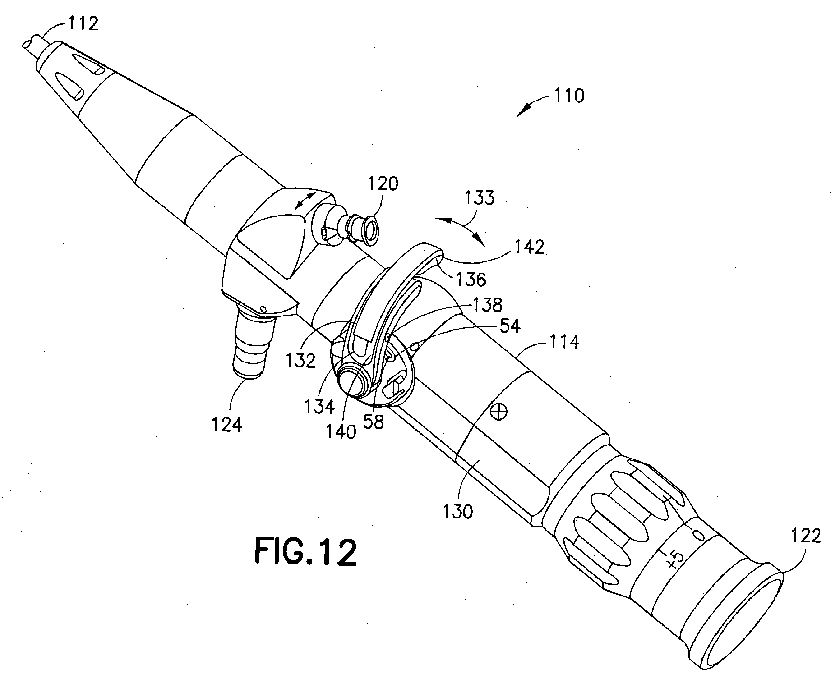

Referring also to Fig. 12, another example embodiment is shown. In this example the endoscope 110 comprises a shaft 112 and a main section 114 which forms a handle. The shaft 112 has a front or distal end which is controllably deflectable. The distal end is adapted to deflection in up and down directions. However, any suitable deflection capability could be provided.

The main section 114 is located at the proximal end of the shaft 112. The main section 114 has a working instrument/irrigation inlet 120, an eyepiece 122, and a fiber optics connector 124. Deflection control wires or cables extend from the distal end to a pulley in the main section 114. The pulley is rotatably mounted in the frame 130 of the main section 114.

The pulley and deflection control wires form part of a deflection control for a user to control deflection of the deflectable distal end from the main section 114. The deflection control also comprises a lever assembly 132. The lever assembly 132 is connected to the pulley to axially rotate the pulley when the lever assembly 132 is rotated forwards or backwards as indicated by arrows 133. The lever assembly 132 comprises a control lever 134 and a lock lever 136. The control lever 134 is fixedly connected to the pulley. The lock lever 136 is pivotably connected to the control lever 134 by a pivot 138. Thus, the lock lever 136 can pivot on the control lever 134, and the lock lever 136 is connected to the pulley by the control lever 134; in series. However, any suitable movable connection of the lock lever on the control lever could be provided.

The lock lever 136 has a first end 140 and a second end 142 on opposite sides of the pivot pin 138. The second end 142 forms a finger contact area. The first end 140 has a locking section on an inward facing side, such as a projection for example. In this embodiment the locking section is a pin attached to the first end 140. However, any suitable locking section could be provided. A spring is provided between the control lever and the lock lever to bias the second end 142 outward and the first end 140 inward on the control lever 134. However, a user can use his/her thumb to depress the second end 142 to thereby pivot the lock lever 136 at the pivot 138 and move the first end 140 outward.

The main section 114 includes a locking section or pad 54 located opposite the locking section of the lock lever 136. In this embodiment the pad 54 is an insert which is fixedly inserted into a receiving area of the main section. The insert has a general curved or arched shape. However, in an alternate embodiment the pad could be integrally formed with the frame of the main section, or any suitably sized and shaped locking section (for engaging with the locking section of the lock lever) could be provided. The pad 54 has a series of recesses 58. The recesses 58 are sized, shaped and arranged in an arch or curve to receive the bottom tip of the locking projection of the lock lever at different angular positions of the lever assembly 132. The recesses 58 allow positive location of the tip and, thus, positive location of the angular position of the lever assembly 32, at one of a plurality of fixed predetermined locations selected by the user. When the lock lever 136 is at its home position with the second end 142 biased outward by the spring, the tip is biased into one of the recesses 58. This prevents the lever assembly 132 from rotating relative to the main section 114 and, thus, prevents the lever assembly 132 from rotating the pulley. This forms a lock or brake for the deflection control system; fixing the position or shape of the distal end on the shaft 112. The projection from the first end 134 of the lock lever forms a first lock/brake section which engages with a second lock/brake section formed by the pad 54.

With the same hand holding the handle formed by the main section 114, the user can use his or her finger to depress the second end 142 of the lock lever 136 inward to thereby disengage the projection from the first end 140 of the lock lever from the lock pad 54. The user is then free to swing the lever assembly 132 (both levers 134, 136 moving together in unison) forward or backwards as indicated by arrows 133. Once a desired deflection of the distal end of the shaft is obtained, the user can release the second end 142 of the lock lever 136 and the spring can automatically biased the lock lever 136 back to its home position. This automatically locks the lever assembly at a fixed angular position on the main section 114 due to the re-engagement of the lock/brake sections on the lock lever first end and the main section.

In this example embodiment the endoscope main section is provided with a vertically oriented pulley, and the control/lock lever assembly rotates about the longitudinal axis of the endoscope main section. This assembly proximal portion is located on the upper side (or alternatively the lower side) of the endoscope main section. Multiple recesses may be provided on either side of the main section.

A single finger of a user can be used to unlock the locking engagement with the multiple recesses 58 by pushing the second end of the lock lever towards to the main section, and then rotating the deflection control wire pulley with the same finger by pushing the control/lock lever proximal section forward or backward. The locking engagement can then be reengaged by releasing the user finger from the control/lock lever proximal section.

Moving the control/lock lever assembly in this way enables more intuitive controls of the deflection at the distal tip of the flexible shaft of the endoscope. It may provide greater ease of use for the end user and can also be thought of as a change in design choice. For example, when the user desires to deflect the distal tip of the endoscope in an up/down configuration, it may be considered more intuitive if the lever is moved forward and backward.

When the user desires to deflect the distal tip of the endoscope from side to side, it may be considered more intuitive to move the lever left and right (see Figs. 1-2). The way in which the control wires are connected from the distal tip of the flexible shaft of the endoscope into the control/lock lever assembly will ultimately determine how deflection occurs at the distal tip of the flexible shaft of the endoscope.

## Claims

1. An endoscope comprising:
a shaft (12) having a deflectable distal end;
a main section (14) at a proximal end of the shaft, where the
main section forms a handle of the endoscope; and
a deflection control (26, 28, 32) connected to the distal end, wherein
the deflection control comprises a combined lock and deflection control member at the main section comprising a control lever (34) pivotably connected to the main section at a pivot and extending away from the pivot in a general cantilever fashion, and a lock lever (36) pivotably connected to the control lever, where a first end of the lock lever is configured to contact the main section and where a second end of the lock lever is configured to be pressed inward on the control lever towards the main section to pivot the first end out of contact with the main section,
**characterized in that** the first end of the lock lever comprises a projection (46) adapted to contact a lock section (54) on the main section when the lock lever is at a locked position, and wherein the projection is disengaged from the lock section on the main section when the lock lever is pivoted on the control lever to an unlocked position.

2. An endoscope as in claim 1 wherein the combined lock and deflection control member is adapted to be moved to a selected location by a single finger of a user and thereby deflect the distal end, and wherein the combined lock and deflection control member is adapted to be locked at the user selected location by movement of the lock lever to a locked position by the single finger of the user.

3. An endoscope as in claim 1 wherein the control lever is connected to a control wire pulley of the deflection control, wherein the control wire pulley is connected to the deflectable distal end.

4. An endoscope as in claim 1 wherein the lock lever is biased by a spring on the control lever towards a locked position with the main section.

5. An endoscope as in claim 1 wherein the lock section on the main section comprises a curved insert mounted onto the main section.

6. An endoscope as in claim 1 wherein the lock section on the main section comprises recesses at predetermined locations along a curved path.

7. An endoscope as in claim 1 wherein a first section of the control lever is located at a first exterior side of the main section and a second section of the control lever is located at an adjacent second exterior side of the handle, where the control lever has a cantilevered curved shape over the first and second exterior sides.

8. An endoscope comprising:
a shaft (112) having a deflectable distal end;
a handle (114) at a proximal end of the shaft; and
a deflection control connected to the distal end, wherein the deflection control comprises:
a control lever (134) pivotably connected to a first side
of the handle, where the control lever extends from the first side of the handle in a general cantilever fashion, over an adjacent second side of the handle; and
a lock lever (136) pivotably connected to the control lever, wherein the lock lever comprises a lock section adapted to contact a lock section (54) on the handle,
wherein the lock lever is pivotably connected to the control lever by a pivot, **characterized in that** the lock lever includes a first end (140) and a second end (142) on opposite sides of the pivot, where the first end comprises the lock section of the lock lever, where the second end comprises a finger contact area for a user to depress the second end and pivotably rotate the lock lever on the control lever, and wherein the lock section of the lock lever is disengaged from the lock section on the handle when the lock lever is moved on the control lever to an unlocked position.

9. An endoscope as in claim 8 wherein the control lever is connected to a control wire pulley of the deflection control, wherein the control wire pulley is connected to the deflectable distal end by a control wire.

10. An endoscope as in claim 8 wherein the lock lever is biased by a spring on the control lever towards a locked position with the handle.

11. An endoscope as in claim 8 wherein the lock section on the handle comprises a curved insert mounted onto the handle.

12. An endoscope as in claim 8 wherein the lock section on the handle comprises recesses at predetermined locations along a curved path.

13. An endoscope as in claim 8 where the control lever has a longitudinally curved shape and where the lock lever has a longitudinally curved shape generally in a same direction of curvature as the control lever.

14. A method of operating an endoscope comprising:
rotating a lock lever in a first direction from a first locked position to a second unlocked position, where the lock lever is pivotably connected to a control lever, where the control lever is pivotably connected to a control wire pulley of a deflection control system of the endoscope; and
after the lock lever is moved to the second unlocked position, moving the lock lever and the control lever together in a second different direction to thereby rotate the control wire pulley.

15. A method as in claim 14 further comprising, after the lock lever and control lever are rotated in the second direction to a user selected location, allowing a spring to pivot the lock lever on the control lever in a third direction opposite to the first direction such that a lock section of the lock lever engages a lock section on a handle section of the endoscope.

## Patentansprüche

1. Endoskop, umfassend:
einen Schaft (12) mit einem ablenkbaren distalen Ende;
einen Hauptabschnitt (14) an einem proximalen Ende des Schafts, wobei der Hauptabschnitt einen Griff des Endoskops bildet; und
eine Ablenksteuerung (26, 28, 32), die mit dem distalen Ende verbunden ist, wobei
die Ablenksteuerung ein kombiniertes Verriegelungs- und Ablenksteuerungsglied an dem Hauptabschnitt umfasst, umfassend einen Steuerungshebel (34) der drehbar mit dem Hauptabschnitt an einem Drehpunkt verbunden ist und sich von dem Drehpunkt in einer allgemeinen Freiträger-Weise weg erstreckt, und einen Verriegelungshebel (36), der drehbar mit dem Steuerungshebel verbunden ist, wobei ein erstes Ende des Verriegelungshebels konfiguriert ist, den Hauptabschnitt zu berühren, und wobei ein zweites Ende des Verriegelungshebels konfiguriert ist, nach innen auf den Steuerungshebel gedrückt zu werden, hin zu dem Hauptabschnitt, um das erste Ende aus der Berührung mit dem Hauptabschnitt herauszudrehen,
**dadurch gekennzeichnet, dass**
das erste Ende des Verriegelungshebels einen Vorsprung (46) umfasst, der angepasst ist, einen Verriegelungsabschnitt (54) auf dem Hauptabschnitt zu berühren, wenn sich der Verriegelungshebel in einer verriegelten Position befindet, und wobei der Vorsprung von dem Verriegelungsabschnitt auf dem Hauptabschnitt gelöst ist, wenn der Verriegelungshebel auf dem Steuerungshebel in eine nicht-verriegelte Position gedreht ist.

2. Endoskop nach Anspruch 1, wobei das kombinierte Verriegelungs- und Ablenksteuerungsglied angepasst ist, in eine gewählte Position durch einen einzelnen Finger eines Benutzers bewegt zu werden und dadurch das distale Ende abzulenken, und wobei das kombinierte Verriegelungs- und Ablenksteuerungsglied angepasst ist, an der von dem Benutzer gewählten Position verriegelt zu werden, durch Bewegung des Verriegelungshebels zu einer verriegelten Position mit dem einzelnen Finger des Benutzers.

3. Endoskop nach Anspruch 1, wobei der Steuerungshebel mit einer Steuerungsleitungsrolle der Ablenksteuerung verbunden ist, wobei die Steuerungsleitungsrolle mit dem ablenkbaren distalen Ende verbunden ist.

4. Endoskop nach Anspruch 1, wobei der Verriegelungshebel durch eine Feder auf dem Steuerungshebel hin zu einer verriegelten Position mit dem Hauptabschnitt vorgespannt ist.

5. Endoskop nach Anspruch 1, wobei der Verriegelungsabschnitt auf dem Hauptabschnitt eine gekrümmte Einlage umfasst, die auf dem Hauptabschnitt angebracht ist.

6. Endoskop nach Anspruch 1, wobei der Verriegelungsabschnitt auf dem Hauptabschnitt Aussparungen an vorbestimmten Positionen entlang einer Kurvenbahn umfasst.

7. Endoskop nach Anspruch 1, wobei sich ein erster Abschnitt des Steuerungshebels auf einer ersten Außenseite des Hauptabschnitts befindet und sich ein zweiter Abschnitt des Steuerungshebels auf einer benachbarten zweiten Außenseite des Griffs befindet, wobei der Steuerungshebel eine freitragende gebogene Form über der ersten und der zweiten Außenseite aufweist.

8. Endoskop, umfassend:
einen Schaft (112) mit einem ablenkbaren distalen Ende;
einen Griff (114) an einem proximalen Ende des Schafts; und
eine Ablenksteuerung, die mit dem distalen Ende verbunden ist, wobei die Ablenksteuerung umfasst:
einen Steuerungshebel (134), der drehbar an einer ersten Seite des Griffs verbunden ist, wobei sich der Steuerungshebel von der ersten Seite des Griffs in einer allgemeinen Freiträger-Weise erstreckt, über eine benachbarte zweite Seite des Griffs; und
einen Verriegelungshebel (136), der drehbar an dem Steuerungshebel verbunden ist, wobei der Verriegelungshebel einen Verriegelungsabschnitt umfasst, der angepasst ist, einen Verriegelungsabschnitt (54) auf dem Griff zu berühren,
wobei der Verriegelungshebel drehbar mit dem Steuerungshebel durch einen Drehpunkt verbunden ist, **dadurch gekennzeichnet, dass** der Verriegelungshebel ein erstes Ende (140) und ein zweites Ende (142) auf entgegengesetzten Seiten des Drehpunkts umfasst, wobei das erste Ende den Verriegelungsabschnitt des Verriegelungshebels umfasst, wobei das zweite Ende einen Fingerberührungsbereich für einen Benutzer umfasst, um das zweite Ende hinunter zu drücken und drehbar den Verriegelungshebel auf dem Steuerungshebel zu drehen und wobei der Verriegelungsabschnitt des Verriegelungshebels von dem Verriegelungsabschnitt auf dem Griff gelöst ist, wenn der Verriegelungshebel auf dem Steuerungshebel in eine nicht-verriegelte Position bewegt ist.

9. Endoskop nach Anspruch 8, wobei der Steuerungshebel mit einer Steuerungsleitungsrolle der Ablenksteuerung verbunden ist, wobei die Steuerungsleitungsrolle durch eine Steuerungsleitung mit dem ablenkbaren distalen Ende verbunden ist.

10. Endoskop nach Anspruch 8, wobei der Verriegelungshebel durch eine Feder auf dem Steuerungshebel hin zu einer verriegelten Position mit dem Griff vorgespannt ist.

11. Endoskop nach Anspruch 8, wobei der Verriegelungsabschnitt auf dem Griff eine gekrümmte Einlage umfasst, die auf dem Griff angebracht ist.

12. Endoskop nach Anspruch 8, wobei der Verriegelungsabschnitt auf dem Griff Aussparungen an vorbestimmten Positionen entlang einer Kurvenbahn umfasst.

13. Endoskop nach Anspruch 8, wobei der Steuerungshebel eine länglich gebogene Form aufweist und wobei der Verriegelungshebel eine länglich gebogene Form aufweist im Wesentlichen in derselben Krümmungsrichtung wie der Steuerungshebel.

14. Verfahren zum Betrieb eines Endoskops, umfassend:
Rotieren eines Verriegelungshebels in einer ersten Richtung von einer ersten verriegelten Position zu einer zweiten nicht-verriegelten Position, wobei der Verriegelungshebel drehbar mit einem Steuerungshebel verbunden ist, wobei der Steuerungshebel drehbar mit einer Steuerungsleitungsrolle eines Ablenksteuerungssystems des Endoskops verbunden ist; und
nachdem der Verriegelungshebel zu der zweiten nicht-verriegelten Position bewegt wird, Bewegen des Verriegelungshebels und des Steuerungshebels zusammen in eine zweite, andere Richtung, um dadurch die Steuerungsleitungsrolle zu rotieren.

15. Verfahren nach Anspruch 14, ferner umfassend, nachdem der Verriegelungshebel und Steuerungshebel in der zweiten Richtung zu einer Benutzer-gewählten Position gedreht werden, Ermöglichen einer Feder, den Verriegelungshebel auf dem Steuerungshebel in eine dritte Richtung entgegensetzt zu der ersten Richtung zu drehen, so dass ein Verriegelungsabschnitt des Verriegelungshebels in einen Verriegelungsabschnitt auf einem Griffabschnitt des Endoskops eingreift.

## Revendications

1. Endoscope comprenant:
un arbre (12) ayant une extrémité distale pouvant être défléchie;
une section principale (14) au niveau d'une extrémité proximale de l'arbre, dans lequel la section principale forme une poignée de l'endoscope ; et
une commande de déflection (26, 28, 32) connectée à l'extrémité distale, dans lequel
la commande de déflection comprend un élément de commande de verrouillage et de déflection combiné au niveau de la section principale comprenant un levier de commande (34) connecté de façon pivotante à la section principale au niveau d'un pivot et s'étendant à distance du pivot d'une manière générale en porte à faux, et un levier de verrouillage (36) connecté de façon pivotante au levier de commande, dans lequel une première extrémité du levier de verrouillage est configurée de manière à entrer en contact avec la section principale et dans lequel une seconde extrémité du levier de verrouillage est configurée pour être pressée vers l'intérieur sur le levier de commande en direction la section principale pour faire pivoter la première extrémité hors de contact avec la section principale, **caractérisé en ce que**
la première extrémité du levier de verrouillage comprend une saillie (46) adaptée pour entrer en contact avec une section de verrouillage (54) sur la section principale lorsque le levier de verrouillage est dans une position de verrouillage, et dans lequel la saillie est désengagée de la section de verrouillage sur la section principale, lorsque le levier de verrouillage est pivoté sur le levier de commande vers une position de déverrouillage.

2. Endoscope selon la revendication 1, dans lequel l'élément de commande de verrouillage et de déflection combiné est adapté pour être déplacé vers un emplacement sélectionné au moyen d'un seul doigt d'un utilisateur et défléchir ainsi l'extrémité distale, et dans lequel l'élément de commande de verrouillage et de déflection combiné est adapté pour être verrouillé au niveau de l'emplacement choisi par l'utilisateur par le déplacement du levier de verrouillage vers une position de verrouillage au moyen du seul doigt de l'utilisateur.

3. Endoscope selon la revendication 1, dans lequel le levier de commande est relié à une poulie de câble de commande de la commande de déflection, dans lequel la poulie de câble de commande est reliée à l'extrémité distale pouvant être défléchie.

4. Endoscope selon la revendication 1, dans lequel le levier de verrouillage est sollicité par un ressort sur le levier de commande vers une position de verrouillage avec la section principale.

5. Endoscope selon la revendication 1, dans lequel la section de verrouillage sur la section principale comprend un insert incurvé monté sur la section principale.

6. Endoscope selon la revendication 1, dans lequel la section de verrouillage sur la section principale comporte des évidements au niveau d'emplacements prédéterminés le long d'une trajectoire courbe.

7. Endoscope selon la revendication 1, dans lequel une première section du levier de commande est située au niveau d'un premier côté extérieur de la section principale et une seconde section du levier de commande est située au niveau d'un second côté extérieure de la poignée adjacent, dans lequel le levier de commande présente une forme incurvée en porte à faux sur les premier et second côtés extérieurs.

8. Endoscope comprenant:
un arbre (112) ayant une extrémité distale pouvant être défléchie;
une poignée (114) au niveau d'une extrémité proximale de l'arbre; et
une commande de déflection connectée à l'extrémité distale, dans lequel la commande de déflection comprend:
un levier de commande (134) connecté de façon pivotante à un premier côté de la poignée, dans lequel le levier de commande s'étend à partir du premier côté de la poignée d'une manière générale en porte à faux, sur un second côté adjacent de la poignée ; et
un levier de verrouillage (136) connecté de façon pivotante pivoter au levier de commande, dans lequel le levier de verrouillage comprend une section de verrouillage adaptée pour entrer en contact avec une section de verrouillage (54) sur la poignée,
dans lequel le levier de verrouillage est relié de façon pivotante au levier de commande par un pivot, **caractérisé en ce que** le levier de verrouillage comprend une première extrémité (140) et une seconde extrémité (142) sur des côtés opposés du pivot, dans lequel la première extrémité comprend la section de verrouillage du levier de verrouillage, dans lequel la seconde extrémité comprend une zone de contact pour le doigt pour permettre à un utilisateur d'enfoncer sur la seconde extrémité et de faire tourner par pivotement le levier de verrouillage sur le levier de commande, et dans lequel la section de verrouillage sur levier de verrouillage est désengagée de la section de verrouillage sur la poignée lorsque le levier de verrouillage est déplacé sur le levier de commande vers une position de déverrouillage.

9. Endoscope selon la revendication 8, dans lequel le levier de commande est relié à une poulie de câble de commande de la commande de déflection, dans lequel la poulie de câble de commande est reliée à l'extrémité distale pouvant être défléchie par un câble de commande.

10. Endoscope selon la revendication 8, dans lequel le levier de verrouillage est sollicité par un ressort sur le levier de commande en direction d'une position de verrouillage avec la poignée.

11. Endoscope selon la revendication 8, dans lequel la section de verrouillage sur la poignée comprend un insert incurvé monté sur la poignée.

12. Endoscope selon la revendication 8, dans lequel la section de verrouillage sur la poignée comprend des évidements au niveau d'emplacements prédéterminés le long d'une trajectoire courbe.

13. Endoscope selon la revendication 8, dans lequel le levier de commande présente une forme incurvée longitudinalement et dans lequel le levier de verrouillage présente une forme incurvée longitudinalement globalement dans un même sens de courbure que le levier de commande.

14. Procédé de fonctionnement d'un endoscope, comprenant:
la rotation d'un levier de verrouillage dans un premier sens à partir d'une première position de verrouillage jusqu'à une seconde position de déverrouillage, dans laquelle le levier de verrouillage est relié de façon pivotante à un levier de commande, dans lequel le levier de commande est connecté de façon pivotante à une poulie de câble de commande d'un système de commande de déflection endoscope; et
après que le levier de verrouillage ait été déplacé vers la seconde position de déverrouillage, le déplacement du levier de verrouillage et du levier de commande ensemble dans un second sens différent pour faire tourner ainsi la poulie de câble de commande.

15. Procédé selon la revendication 14, comprenant en outre, après que le levier de verrouillage et le levier de commande ait été mis en rotation dans le second sens jusqu'à un emplacement sélectionné par l'utilisateur, l'étape consistant à permettre à un ressort de pivoter le levier de verrouillage sur le levier de commande dans un troisième sens opposé au premier sens de telle sorte qu'une section de verrouillage du levier de verrouillage vient en prise avec une section de verrouillage sur une section de poignée de l'endoscope.
